# EUROPEAN PATENT SPECIFICATION

(11) **EP 4 344 413 B1**
(45) Date of publication and mention of the grant of the patent: **28.05.2025**
(21) Application number: 22776978.3
(22) Date of filing: 20.07.2022
(51) Int. Cl.: A61N 1/04, A61N 1/36, A61N 1/40

(54) **CONDUCTIVE PAD GENERATING TUMOR TREATING FIELD**
LEITFÄHIGES PAD ZUR ERZEUGUNG EINES TUMORBEHANDLUNGSFELDES
TAMPON CONDUCTEUR GÉNÉRANT UN CHAMP DE TRAITEMENT DE TUMEUR

(30) Priority: 21.07.2021 US 202163224241 P
(43) Date of publication of application: 03.04.2024
(60) Divisional application: 25171829.2
(73) Proprietor: Novocure GmbH, 6340 Baar (CH)
(72) Inventor: DESLAURIERS, Richard, Woodbury, CT 06798 (US)
(74) Representative: Boden, Keith McMurray
(86) International application number: PCT/IB2022/000412
(87) International publication number: WO 2023/002250

(56) References cited:
- WO-A1-2016/201366
- WO-A1-2020/010219
- GB-A- 2 341 104
- US-A1- 2020 155 835

## Description

### BACKGROUND

Tumor Treating Fields (TTFields or TTFs) are low intensity (e.g., 1-3 V/cm) alternating electric fields within the intermediate frequency range (e.g., 50 kHz to 1 MHz, such as 50-500 kHz) that target solid tumors by disrupting mitosis. This non-invasive treatment targets solid tumors and is described, for example, in US Patent Nos. 7,016,725; 7,089,054; 7,333,852; 7,565,205; 8,244,345; 8,715,203; 8,764,675; 10,188,851; and 10,441,776. TTFields are typically delivered through two pairs of transducer arrays that generate perpendicular fields within the treated tumor; the transducer arrays that make up each of these pairs are positioned on opposite sides of the body part that is being treated. More specifically, for the OPTUNE^{®} system, one pair of electrodes of the transducer array is located to the left and right (LR) of the tumor, and the other pair of electrodes of the transducer array is located anterior and posterior (AP) to the tumor. TTFields are approved for the treatment of glioblastoma multiforme (GBM), and may be delivered, for example, via the OPTUNE^{®} system (Novocure Limited, St. Helier, Jersey), which includes transducer arrays placed on the patient's shaved head. More recently, TTFields therapy has been approved as a combination therapy with chemotherapy for malignant pleural mesothelioma (MPM), and may find use in treating tumors in other parts of the body.

Each transducer array used for the delivery of TTFields in the OPTUNE^{®} device comprises a set of non-conductive ceramic disk electrodes, which are coupled to the patient's skin (such as, but not limited to, the patient's shaved head for treatment of GBM) through a layer of conductive medical gel. To form the ceramic disk electrodes, a conductive layer is formed on a top surface of nonconductive ceramic material. A bottom surface of the nonconductive ceramic material is coupled to the conductive medical gel. The nonconductive ceramic material is a safety feature to ensure that direct-current signals are blocked from unintentionally being transmitted to the patient by mistake.

By interposing a nonconductive ceramic material between the conductive layer and the conductive medical gel, the prior art system was thought to ensure the patient remains protected. The purpose of the medical gel is to deform to match the body's contours and to provide good electrical contact between the arrays and the skin; as such, the gel interface bridges the skin and reduces interference. The device is intended to be continuously worn by the patient for 2-4 days before removal for hygienic care and re-shaving (if necessary), followed by reapplication with a new set of arrays. As such, the medical gel remains in substantially continuous contact with an area of the patient's skin for a period of 2-4 days at a time, and there is only a brief period of time in which the area of skin is uncovered and exposed to the environment before more medical gel is applied thereto.

One approach to applying the TTField in different directions is to apply the field between a first set of electrodes for a period of time, then applying a field between a second set of electrodes for a period of time, then repeating that cycle for an extended duration (e.g., over a period of days or weeks).

In order to generate the TTFields, current is applied to each electrode of the transducer array. The application of current over a period of time causes each electrode to warm and eventually become hot, and thus uncomfortable or painful to the patient. In order to maintain the desired temperature of the transducer array, the current applied is lowered, resulting in a weaker TTField, or the transducer array is powered off, thus shortening the duration of treatment. Additionally, the prior art teaches electrodes made from rigid and/or inflexible materials, such as ceramics, which do not contour to the patient.

Because of this heating of the transducer array, new and improved array assemblies that reduce the temperature of the transducer array while generating a more powerful TTField are desired. It is to such assemblies and methods of producing and using the same, that the present disclosure is directed.

WO-A-2016/201366 discloses an electrode of a monitoring and/or therapy unit which is provided at and/or around the skin interface by a porous material, such as a foam polymer, that is impregnated with conductive gel.

US-A-2020/0155835 discloses transducer assemblies which include a plurality of capacitively-coupled electrode elements mounted on a support, with the electrode elements being configured for placement against a subject's body, preferably with a layer of hydrogel disposed on the surface of the electrode elements that faces the subject's body, and the support holds the electrode elements against the subject's body.

### SUMMARY OF THE INVENTION

The problem of reducing the temperature of the transducer array while generating a more powerful TTField is solved by a system for delivering TTFields to a body of a subject according to claim 1.

Embodiments of the invention are disclosed in the dependent claims.

The details of one or more implementations of the subject matter described in this specification are set forth in the accompanying drawings and the description below. Other aspects, features and advantages of the subject matter will become apparent from the description, the drawings, and the claims.

### BRIEF DESCRIPTION OF THE DRAWINGS

The accompanying drawings, which are incorporated in and constitute a part of this specification, illustrate one or more implementations described herein and, together with the description, explain these implementations. The drawings are not intended to be drawn to scale, and certain features and certain views of the figures may be shown exaggerated, to scale or in schematic in the interest of clarity and conciseness. Not every component may be labeled in every drawing. Like reference numerals in the figures may represent and refer to the same or similar element or function. In the drawings:
FIG. 1 is an exemplary embodiment of a schematic diagram of electrodes as applied to living tissue.
FIG. 2 is an exemplary embodiment of an electronic device configured to generate a TTField constructed in accordance with the present disclosure.
FIG. 3 is a block diagram of an exemplary embodiment of a pad constructed in accordance with the present disclosure.
FIG. 4 is a block diagram of another exemplary embodiment of a pad constructed in accordance with the present disclosure.
FIG. 5 is a cross section of an exemplary embodiment of an array assembly constructed in accordance with the present disclosure.
FIG. 6 is a cross-sectional view of an exemplary embodiment of an electrode element constructed in accordance with the present disclosure.
FIG. 7 is a top view of an exemplary embodiment of a fabric layer constructed in accordance with the present disclosure.
FIG. 8 is a cross-sectional diagram of an exemplary embodiment of a foam layer constructed in accordance with the present disclosure.
FIG. 9 is a cross section of another exemplary embodiment of an array assembly constructed in accordance with the present disclosure.
FIG. 10 is a cross-sectional diagram of an exemplary embodiment of a pad constructed in accordance with the present disclosure.
FIG. 11 is a top view of an exemplary embodiment of a pad constructed in accordance with the present disclosure.
FIG. 12 is a process flow diagram of an exemplary embodiment of a process of using the electronic apparatus to apply a TTField to a patient.

### DETAILED DESCRIPTION

Before explaining at least one embodiment of the inventive concept(s) in detail by way of exemplary language and results, it is to be understood that the inventive concept(s) is not limited in its application to the details of construction and the arrangement of the components set forth in the following description. The inventive concept(s) is capable of other embodiments or of being practiced or carried out in various ways. As such, the language used herein is intended to be given the broadest possible scope and meaning; and the embodiments are meant to be exemplary - not exhaustive. Also, it is to be understood that the phraseology and terminology employed herein is for the purpose of description and should not be regarded as limiting.

Headings are provided for convenience only and are not to be construed to limit the invention in any manner. Embodiments illustrated under any heading or in any portion of the disclosure may be combined with embodiments illustrated under the same or any other heading or other portion of the disclosure. Any combination of the elements described herein in all possible variations thereof is encompassed by the invention unless otherwise indicated herein or otherwise clearly contradicted by context.

Unless otherwise required by context, singular terms shall include pluralities and plural terms shall include the singular.

All of the compositions, assemblies, systems, kits, and/or methods disclosed herein can be made and executed without undue experimentation in light of the present disclosure. Where a method claim does not specifically state in the claims or description that the steps are to be limited to a specific order, it is no way intended that an order be inferred, in any respect. This holds for any possible non-express basis for interpretation, including matters of logic with respect to arrangement of steps or operational flow, plain meaning derived from grammatical organization or punctuation, or the number or type of embodiments described in the specification.

As utilized in accordance with the present disclosure, the following terms, unless otherwise indicated, shall be understood to have the following meanings:
The use of the term "a" or "an" when used in conjunction with the term "comprising" in the claims and/or the specification may mean "one," but it is also consistent with the meaning of "one or more," "at least one," and "one or more than one." The term "plurality" refers to "two or more."

In addition, the use of the term "at least one of X, Y, and Z" will be understood to include X alone, Y alone, and Z alone, as well as any combination of X, Y, and Z. The use of ordinal number terminology (e.g., "first," "second," "third," "fourth," etc.) is solely for the purpose of differentiating between two or more items and is not meant to imply any sequence or order or importance to one item over another or any order of addition, for example.

The use of the term "or" in the claims is used to mean an inclusive "and/or" unless explicitly indicated to refer to alternatives only or unless the alternatives are mutually exclusive.

As used herein, the term TTField (TTFields, or TTF(s)) refers to low intensity (e.g., 1-4 V/cm) alternating electric fields of medium frequencies (about 50 kHz - 1 MHz, and more preferably from about 50 kHz - 500 kHz) that when applied to a conductive medium, such as a human body, via electrodes may be used, for example, to treat tumors as described in US Patent Nos. 7,016,725, 7,089,054, 7,333,852, 7,565,205, 7,805,201, and 8,244,345 by Palti and in a publication by Kirson (see Eilon D. Kirson, et al., Disruption of Cancer Cell Replication by Alternating Electric Fields, Cancer Res. 2004 64:3288-3295). TTFields have been shown to have the capability to specifically affect cancer cells and serve, among other uses, for treating cancer. TTFields therapy is an approved mono-treatment for recurrent glioblastoma (GBM), and an approved combination therapy with chemotherapy for newly diagnosed GBM patients.

As used herein, the term TT Signal is an electrical signal that, when received by electrodes applied to a conductive medium, such as a human body, causes the electrodes to generate the TTField described above. The TT Signal is often an AC electrical signal.

As used herein, the term "pad" refers to one or more conductive materials that is/ are configured to be placed over a part of a body of a subject to generate a TTField upon receiving TT signals from an electric field generator.

Turning now to the inventive concept(s), certain non-limiting embodiments thereof include a system and method of implementing the system, the system comprising an electric field generator configured to generate an electrical signal having an alternating current waveform at a frequency in a range from 50 kHz to 500 kHz; a first conductive lead electrically coupled to the electric field generator, the first conductive lead configured to carry the electrical signal to a pad and/or transducer array electrically coupled to the first conductive lead. Various aspects of the present disclosure are provided in detail below.

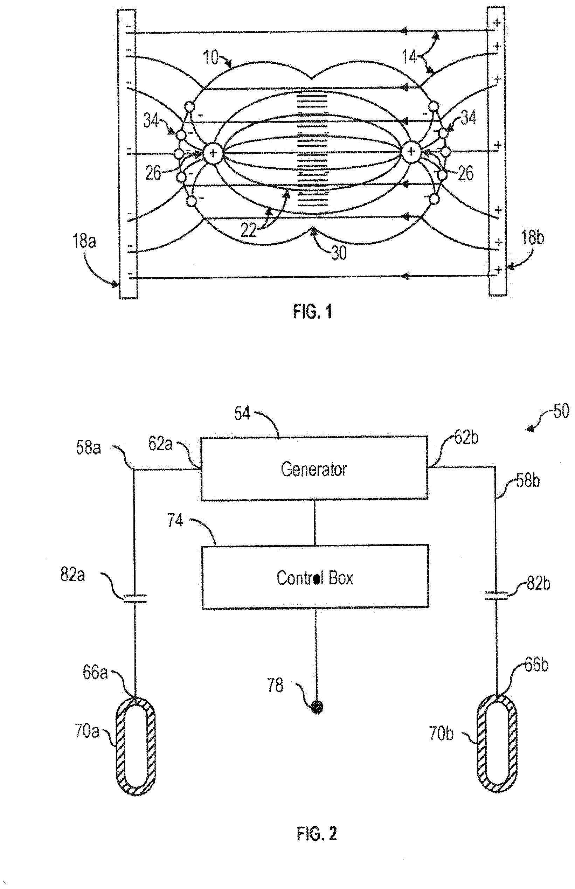

Referring now to the drawings and in particular to FIG. 1, shown therein is an exemplary embodiment of a dividing cell 10, under the influence of external TTFields (e.g., alternating fields in the frequency range of about 100 kHz to about 300 kHz), generally indicated as lines 14, generated by a first electrode 18a having a negative charge and a second electrode 18b having a positive charge. Further shown are microtubules 22 that are known to have a very strong dipole moment. This strong polarization makes the microtubules 22, as well as other polar macromolecules and especially those that have a specific orientation within the cell 10 or its surroundings, susceptible to electric fields. The microtubules 22 positive charges are located at two centrioles 26 while two sets of negative poles are at a center 30 of the dividing cell 10 and point of attachment 34 of the microtubules 22 to the cell membrane. The locations of the charges form sets of double dipoles and therefore are susceptible to electric fields of differing directions. In one embodiment, the cells go through electroporation, that is, DNA or chromosomes are introduced into the cells using a pulse of electricity to briefly open pores in the cell membranes.

Turning now to FIG. 2, the TTFields described above that have been found to advantageously destroy tumor cells may be generated by an electronic apparatus 50. FIG. 2 is a simple schematic diagram of the electronic apparatus 50 illustrating major components thereof. The electronic apparatus 50 includes an electric field generator 54 and a pair of conductive leads 58, including first conductive lead 58a and second conductive lead 58b. The first conductive lead 58a includes a first end 62a and a second end 66a. The second conductive lead 58b includes a first end 62b and a second end 66b. The first end 62a of the first conductive lead 58a is conductively attached to the electric field generator 54 and the first end 62b of the second conductive lead 58b is conductively attached to the electric field generator 54. The electric field generator 54 generates desirable electric signals (TT signals) in the shape of waveforms or trains of pulses as an output. The second end 66a of the first conductive lead 58a is connected to a pad 70a and the second end 66b of the second conductive lead 58b is connected to a pad 70b. Both of the pad 70a and the pad 70b are activated by the electric signals (e.g., TT signals, wave forms). The pad 70a and the pad 70b, being activated by the electric signals, causes an electrical current to flow between the pad 70a and the pad 70b. The electrical current generates an electric field (i.e., TTField), having a frequency and an amplitude, to be generated between the pad 70a and the pad 70b.

While the electronic apparatus 50 shown in FIG. 2 comprises only two pads 70 (the pad 70a and the pad 70b), in some embodiments, the electronic apparatus 50 may comprise more than two pads 70.

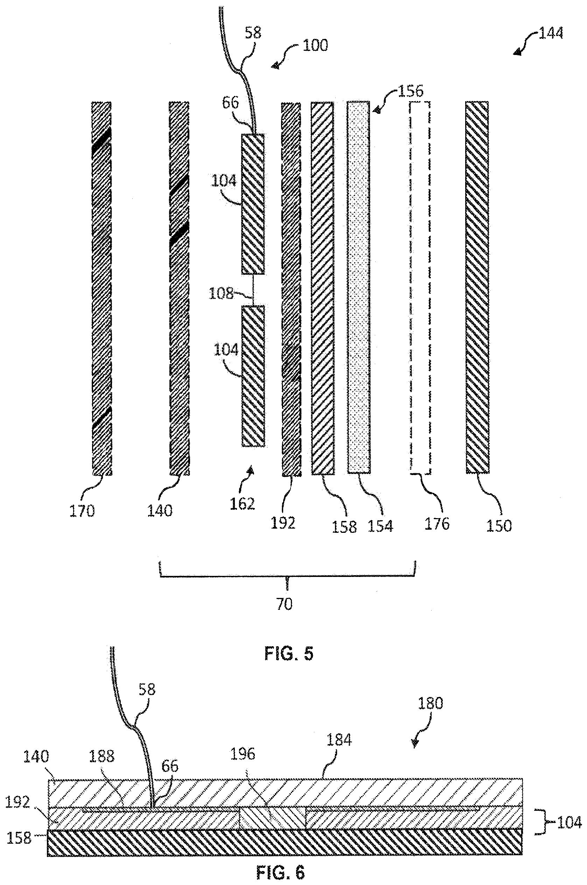

The electric field generator 54 generates an alternating voltage wave form at frequencies in the range from about 50 kHz to about 500 kHz (preferably from about 100 kHz to about 300 kHz) (i.e., the TTFields). The required voltages are such that an electric field intensity in tissue within the treatment area is in the range of about 0.1 V/cm to about 10V/cm. To achieve this field, the potential difference between the two conductors 18 (e.g., the conductor 188 in FIG. 6, or the electrode layer 162 described in detail below, FIG. 5) in each of the pad 70a or the pad 70b is determined by the relative impedances of the system components, e.g., a fraction of the electric field on each component is given by that component's impedance divided by a total circuit impedance.

In certain particular (but non-limiting) embodiments, the pad 70a and the pad 70b generate an alternating electric current and field within a target region of a patient. The target region typically comprises at least one tumor, and the generation of the alternating electric current and field selectively destroys or inhibits growth of the tumor. The alternating electric current and field may be generated at any frequency that selectively destroys or inhibits growth of the tumor, such as at any frequency of a TTField.

In certain particular (but non-limiting) embodiments, the alternating electric current and field may be imposed at two or more different frequencies. When two or more frequencies are present, each frequency is selected from any of the above-referenced values, or a range formed from any of the above-referenced values, or a range that combines two integers that fall between two of the above-referenced values. As used herein, the alternating electric field may be referred to as the electric field or as the TTField.

In order to optimize the electric field (i.e., TTField) distribution, the pad 70a and the pad 70b (pair of pads) may be configured differently depending upon the application in which the pair of pads 70a and 70b are to be used. The pair of pads 70a and 70b, as described herein, are externally applied to a patient, that is, are generally applied to the patient's skin, in order to apply the electric current, and electric field (TTField) thereby generating current within the patient's tissue. Generally, the pair of pads 70a and 70b are placed on the patient's skin by a user such that the electric field is generated across patient tissue within a treatment area. TTFields that are applied externally can be of a local type or widely distributed type, for example, the treatment of skin tumors and treatment of lesions close to the skin surface.

In one embodiment, the user may be a medical professional, such as a doctor, nurse, therapist, or other person acting under the instruction of a doctor, nurse, or therapist. In another embodiment, the user may be the patient, that is, the patient (and/or a helper) may place the pad 70a and the pad 70b on their treatment area.

Optionally, and according to another exemplary embodiment, the electronic apparatus 50 includes a control box 74 and a temperature sensor 78 coupled to the control box 74, which are included to control the amplitude of the electric field so as not to generate excessive heating in the treatment area.

When the control box 74 is included, the control box 74 controls the output of the electric field generator 54, for example, causing the output to remain constant at a value preset by the user. Alternatively, the control box 74 sets the output at the maximal value that does not cause excessive heating of the treatment area. In either of the above cases, the control box 74 may issue a warning, or the like, when a temperature of the treatment area (as sensed by temperature sensor 78) exceeds a preset limit. The temperature sensor 78 may be mechanically connected to and/or otherwise associated with the pad 70a or the pad 70b so as to sense the temperature of the treatment area at either one or both of the pad 70a or the pad 70b. In one embodiment, the control box 74 may turn off, or decrease power of the TT Signal generated by the electrical field generator 54, if a temperature sensed by the temperature sensor 78 meets or exceeds a comfortability threshold. In one embodiment, the comfortability threshold is the temperature at which a patient would be made uncomfortable while using the pad 70a and the pad 70b. In one embodiment, the comfortability threshold is a temperature at or about 40 degrees Celsius. In one embodiment, the comfortability threshold is a temperature of between about 39 degrees Celsius and 42 degrees Celsius, or a specific selected temperature between about 39 degrees Celsius and 42 degrees Celsius.

The conductive leads 58 are standard isolated conductors with a flexible metal shield, preferably grounded thereby preventing spread of any electric field generated by the conductive leads 58. The pad 70a and the pad 70b may have specific shapes and positioning so as to generate the TTField of a desired configuration, direction, and intensity at the treatment area and only at that treatment area so as to focus the treatment.

The specifications of the electronic apparatus 50 as a whole and its individual components are largely influenced by the fact that at the frequency of the TTFields, living systems behave according to their "Ohmic", rather than their dielectric properties.

In one embodiment, to protect the patient from any current due to DC voltage or DC offset voltage passing through the patient, leads 58a and 58b may include a DC blocking component, such as blocking capacitor 82a and blocking capacitor 82b, to block DC current from passing to the pad 70a and the pad 70b. Without being bound by theory, the inventor now believes that the DC blocking component, while important for safety reasons, does not have to be located at the patient interface, i.e., within an electrode of the transducer array, or for that matter, be the non-conductive ceramic disk described above. The blocking capacitors 82a and 82b pass AC voltage to the pad 70a and the pad 70b, and also prevent any DC voltage or DC offset generated by the electric field generator 54 or otherwise present in the electrical signal from passing to or through the patient. DC voltage, when applied to a patient, may have undesirable consequences, such as electrolysis or excessive heating of the pad 70a and pad 70b without the benefit of contributing to the power of the TTField. Thus, the blocking capacitors 82a and 82b can prevent electrolysis due to DC offsets or DC voltage. In one embodiment, the blocking capacitors 82a and 82b are non-polarized capacitors. In one embodiment, the blocking capacitors 82a and 82b have a capacitance of about 1µF. In one embodiment, the blocking capacitor is a "Goldmax, 300 Series, Conformally Coated, X7R Dielectric, 25-250 VDC (Commercial Grade)" leaded non-polarized ceramic capacitor by KEMET Electronics Corporation (Fort Lauderdale, FL, USA).

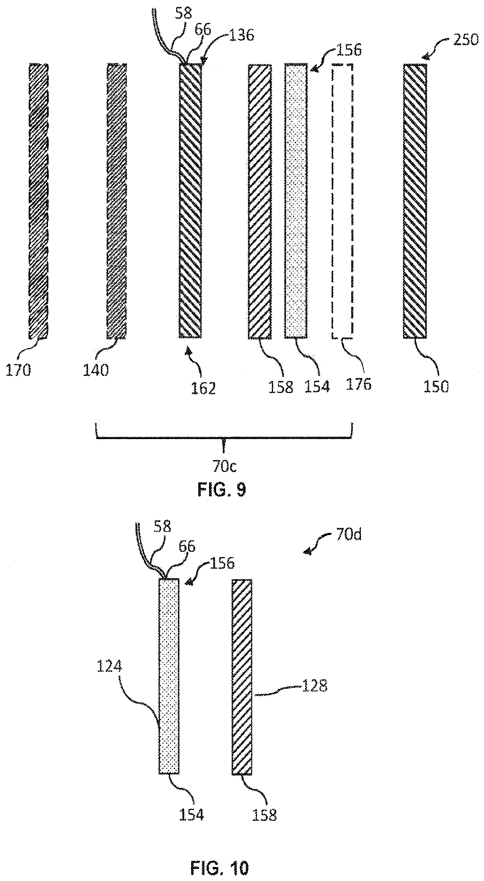

Electrically isolating the patient from the electric field generator 54 may be very important, and so providing the blocking capacitor 82a and/or the blocking capacitor 82b outside of the electric field generator 54 enhances the safety of the patient. The blocking capacitor 82a and the blocking capacitor 82b may be a component of the leads 58a and 58b, or in other embodiments, an additional component at any position between the conductor 188 (see FIG. 6) or electrode element 136 (see FIG. 9) and the electric field generator 54. For example, the blocking capacitors 82a and 82b may be intermediate the first end 62a (or 62b) of the lead 58a (or 58b) and the electric field generator 54, or intermediate the second end 66a (or 66b) of the lead 58a (or 58b) and the pad 70a (or 70b). The inventor believes that the blocking capacitor 82a and 82b can be provided remote from the pad 70a and the pad 70b and still provide for safety of the patient. In other embodiments, the blocking capacitor 82a and the blocking capacitor 82b can be located on a non-patient side of the conductor 188 or electrode element 136.

In other embodiments, the blocking capacitor 82a and the blocking capacitor 82b may be components of the electric field generator 54, that is, the blocking capacitor 82a and the blocking capacitor 82b may be integrated into the electric field generator 54 such that prior to the electrical signal being passed into the leads 58a and 58b, the electrical signal passes through the blocking capacitors 82a and 82b, respectively. Alternatively, the blocking capacitors 82a and 82b may be a component of the pad 70a and the pad 70b, the leads 58a and 58b, or an additional component at any position between a gel layer 158 (see FIG. 10) and the electric field generator 54.

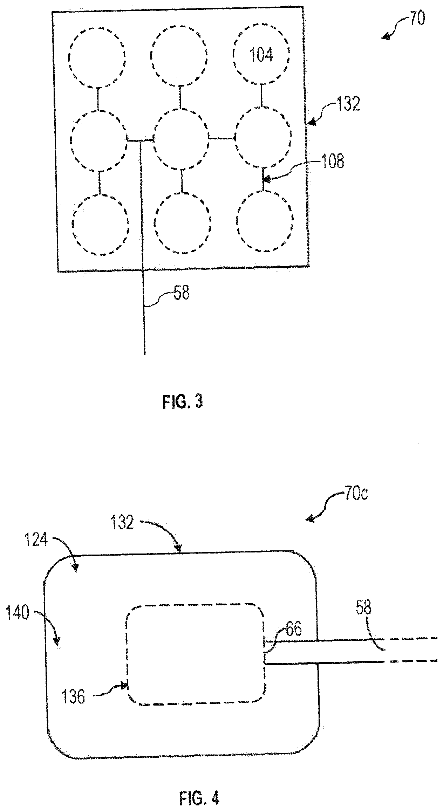

Referring now to FIG. 3, shown therein is a diagram of an exemplary embodiment of the pad 70 constructed in accordance with the present disclosure. The pad 70 includes one or more electrode element 104. As shown in FIG. 3, each pad 70 is configured as a set of one or more electrode elements 104. Pads 70 may utilize electrode elements 104 that are capacitively coupled. In the example shown in FIG. 3, the pad 70 is configured as multiple electrode elements 104 (for example, about 2 cm in diameter) that are interconnected via flex wires 108 (and connected to the electric field generator via the conductive lead 58). Each electrode element 104 may include a ceramic disk and an electrode layer (described below with respect to FIG. 6). In one embodiment, the pad 70 includes an outer peripheral edge 132.

Alternative constructions for the pad 70 may be used, including, for example ceramic elements that are disc-shaped, ceramic elements that are not disc-shaped, and non-ceramic dielectric materials positioned between the electrode layer and a skin-facing surface of the pads 70 over a plurality of flat conductors 188 (see FIG. 6). Examples of non-ceramic dielectric materials positioned over a plurality of flat conductors include: polymer films disposed over pads on a printed circuit board or over flat pieces of metal. Pads 70 that utilize electrode elements 104 that are not capacitively coupled may also be used. In this situation, each electrode element 104 of the transducer array would be implemented using a region of a conductive material that is configured for placement against a person's body, with no insulating dielectric layer disposed between the electrode elements 104 and the body. Examples of the conductive material include a conductive film, a conductive fabric (e.g., fabric layer 150, see FIG. 5), and a conductive foam (e.g., foam layer 154, see FIG. 5). Other alternative constructions for implementing the pads 70 may also be used, as long as they are capable of delivering TTFields to the person's body. Optionally, a gel layer 158 may be disposed between the pad 70 and the person's body in any of the embodiments described herein (see FIG. 6).

Referring now to FIG. 4, shown therein is a top plan view of an exemplary embodiment of a pad 70c. The pad 70c is an exemplary embodiment of the pad 70a or the pad 70b. The pad 70c may be provided with a top 124, a bottom 128 (shown in FIG. 10 for the pad 70d), an outer peripheral edge 132, and an electrode element 136 bounded by the outer peripheral edge 132. As shown, the pad 70c is connected to the second end 66 of the conductive lead 58. The pad 70c is constructed so as to have sufficient flexibility and to be able to conform to a portion of the patient, such as a portion of the patient's head, the patient's knee, the patient's elbow, or the like. The pad 70c may also be constructed such that the electrode element 136 is continuous, and extends to the outer peripheral edge 132. In the example shown, the pad 70c is provided with a rectangular shape, or substantially rectangular shape having rounded vertices. However, it should be understood that the pad 70c can be provided with any type of shape such as a polygon, circle, or fanciful shape. Further, the pad 70c may be constructed such as to be cut and/or shaped at a point of use so as to be custom fitted for a particular part of a particular patient.

In one embodiment, the pad 70c is provided with a durable topcoat layer 140 as the top 124. The durable topcoat layer 140 may be a non-woven, non-conductive fabric. The durable topcoat layer 140 provides a safe handling surface for the pad 70c to electrically isolate the electrode element 136 from the top 124 of the pad 70c. In some embodiments, the durable topcoat layer 140 is colored to match or approximate the skin color of the patient.

In one embodiment, the durable topcoat layer 140 may be "breathable", that is, the durable topcoat layer 140 includes one or more perforation or the like extending from the top 124 to the bottom 128 to enable air-flow to other layers of the pad 70c as described below. The one or more perforation may have the same or different dimension(s) as one or more other perforation, as well as the same or different shape as one or more other perforation.

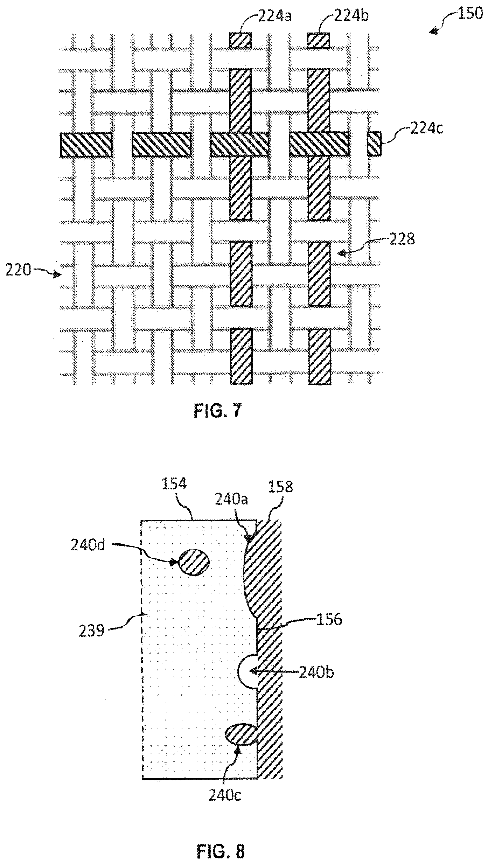

Referring now to FIG. 5, shown therein is a cross section of an exemplary embodiment of an array assembly 144 constructed in accordance with the present disclosure. The array assembly 144 generally comprises one or more layer, including a fabric layer 150, a foam layer 154, a gel layer 158, an electrode layer 162, the durable topcoat layer 140, and a compression layer 170. In one embodiment, the foam layer 154, the gel layer 158, the electrode layer 162, and the durable topcoat layer 140 may, in combination, be referred to as the pad 70. In one embodiment, the fabric layer 150 is a conductive fabric, such as the fabric layer 150 shown in FIG. 7 and discussed in more detail below. In some embodiments, the array assembly 144 includes a dielectric layer 192 disposed between the electrode layer 162 and the gel layer 158.

The foam layer 154 comprises a solid continuous phase material defining a plurality of pockets interspersed throughout the solid continuous phase material. In one embodiment, the solid continuous phase material is made of a conductive material, is attached to a conductive material, or has a conductive material adsorbed onto the solid continuous phase material. In one embodiment, the conductive material is selected from one or more of silver, copper, tin, aluminum, titanium, platinum, carbon, an alloy thereof, and/or some combination thereof. In one embodiment, the foam layer 154 includes a skin-facing surface 156 disposed towards the patient's skin when the pad 70 is in use.

In one embodiment, the skin-facing surface 156 of the foam layer 154 may be in contact with the fabric layer 150. In this embodiment, the fabric layer 150 may cover at least a portion of the skin-facing surface 156 of the foam layer 154. For example, the fabric layer 150 may directly cover at least a portion of the skin-facing surface 156, e.g., the skin-facing surface 156 is in direct contact with the fabric layer 150; however, in other embodiments, the fabric layer 150 may indirectly cover at least a portion of the skin-facing surface 156, e.g., one or more layer of the pad 70 may be disposed between the skin-facing surface 156 and the fabric layer 150, such as, for example, protective layer 176 (FIG. 5).

In one embodiment, the foam layer 154 is a conductive foam. The foam layer 154, being the conductive foam, may have a conductive material attached to the foam layer 154 or have the conductive material adsorbed onto the solid continuous phase of the foam. In one embodiment, the conductive material is selected from one or more of silver, copper, tin, aluminum, titanium, platinum, carbon, an alloy thereof, and/or some combination thereof.

In one embodiment, the foam layer 154 is a silver foam. The silver foam may have a purity of greater than about 99.99% and a porosity of more than about 85%. Exemplary embodiments of the silver foam may include: silver foam, item number MF-AgFom, sold by MTI Corporation (Richmond, CA, USA); SV1972 Silver Foam sold by Stanford Advanced Materials (Lake Forest, CA, USA); Mepilex Ag Molnlycke 278200 sold by MedOnTheGo.com (Alpharetta, GA, USA); Silver Foam Dressing PolyMem MAX manufactured by Ferris Manufacturing (Fort Worth, TX, USA); Ferris PolyMem Silver WIC Silver Cavity Wound Filler manufactured by Ferris Manufacturing; or AQUACEL Ag Foam from ConvaTec (Reading, England, U.K.).

The conductive foam, such as the silver foam, may be selected (in terms of size/area of the foam) and positioned to extend past the outer edges of the electrode elements or past the outer edges of the electrode layer, and may extend to the outer edge of the pad, or beyond. The extra surface area of the conductive foam, both in terms of the planar area that may extend beyond the edges of the electrode elements and in terms of the additional surface area provided by the porous cell structure of the foam, provides a mechanism to dissipate heat from the area of the electrode elements, and thereby reduce the problem of uncomfortable heat on the patient's skin. This in turn allows the use of more powerful TTFields while remaining within the selected temperature comfortability threshold. Alternatively, the advantage of the inventive construct may be realized in reduced time that the TTFields need to be powered down or turned off, and may allow for longer durations of continuous treatment.

In one embodiment, the foam layer 154 is between about 1 mm and about 2 mm thick. In some embodiments, the foam layer 154 may be greater than 2 mm or lesser than 1 mm. The thickness of the foam layer 154 may be selected, for example, based on a desired compressability, flexibility, durability, conductivity, and/or stretchability, or some combination thereof.

In one embodiment, the foam layer 154 has a strong biocompatibility and low reactivity with other layers or components of the array assembly 144. In one embodiment, the foam layer 154 is comprised of an open-cell foam, whereas, in other embodiments, the foam layer 154 is comprised of a closed-cell foam, or varying amounts of both of open-cell foam and closed-cell foam.

In one embodiment, where the dielectric layer 192 is not present, the foam layer 154 is electrically coupled to the electrode layer 162.

In one embodiment, the gel layer 158 may be disposed between the foam layer 154 and the electrode layer 162 (see FIG. 5). In one embodiment, the gel layer 158 includes a gel, such as a conductive gel, a hydrogel, or a conductive hydrogel. In one embodiment, the gel layer 158 is applied to the foam layer 154. The foam layer 154, having the plurality of pockets formed therein, may receive a portion of the gel layer 158 within one or more, or even the majority, of the plurality of the pockets.

In one embodiment, the gel layer 158 is disposed on the skin-facing surface 156 of the foam layer 154. In one embodiment, when the foam layer 154 absorbs or adsorbs the gel layer 158, the gel layer 158 may be considered to be on both the skin-facing surface 156 and the opposite side of the foam layer 154.

In one embodiment, the gel layer 158 is between about 10 thousandths of an inch (10 mils or 0.254 mm) and 20 thousandths of an inch (20 mils or 0.508 mm) thick. In one embodiment, the gel layer 158 is in contact with the foam layer 154 and may be polymerized while in contact with the foam layer 154. In one embodiment, the gel layer 158 is applied to the foam layer 154 as a liquid hydrogel, which is then cured, or polymerized, to form a semi-solid gel layer 158 on the foam layer 154 and embedded into the plurality of pockets of the foam layer 154.

In one embodiment, the gel layer 158 includes a conductive gel having a bulk electron transport agent providing a source of free ions therein to enable electrical conductivity. In one embodiment, the gel layer 158 is formed primarily of a conductive gel or a semi-solid conductive gel. When present, the source of free ions in the gel may be any salt or other substance that serves as a source of free ions that are capable of floating substantially freely within the gel, wherein the free ions serve to conduct electricity and thus reduce impedance. In one embodiment, the gel layer 158 includes a polymeric hydrogel. In one embodiment, the gel layer 158 has adhesive properties.

The bulk electron transport agent(s) may be any substance that is capable of enhancing the electrical and/or thermal conductivity of the conductive gel. In certain non-limiting embodiments, the bulk electron transport agent(s) includes one or more ionic compounds, one or more metals, or one or more non-metals, as well as any combinations thereof. In certain non-limiting embodiments, the bulk electron transport agent comprises an amorphous carbon and/or a crystalline carbon. Particular (but non-limiting) examples of bulk electron transport agents that may be utilized in accordance with the present disclosure include carbon black, graphene, and graphite.

In one embodiment, the gel layer 158 is formed primarily of a conductive gel or semi-solid conductive gel such as described below. The gel layer 158 may be in any form that allows the array assembly 144 to function in accordance with the present disclosure. The exact thickness of the gel layer 158 is not important so long as the gel layer 158 is of sufficient thickness that the gel layer 158 does not dry out during the treatment. Preferably, the gel layer 158 has high conductivity, is tacky, and is biocompatible for extended periods of time. One suitable gel is AG603 Hydrogel, which is available from AmGel Technologies, 1667 S. Mission Road, Fallbrook, Calif. 92028-4115, USA. The gel layer 158 taught herein may be used with modified hydrogels (which includes not only perforations but also recesses, protrusions, etc.) as disclosed in detail in US Patent Publication No. 2021/0346693 entitled "Conductive Pad Generating Tumor Treating Field and Methods of Production and Use Thereof".

The conductive gel may be in any form that allows the composition to function in accordance with the present disclosure. For example (but not by way of limitation), the conductive gel may be in the form of a hydrogel or a hydrocolloid.

In certain particular (but non-limiting) embodiments, the conductive gel is sterile. In addition, in certain non-limiting embodiments, the conductive gel will not substantially degrade upon exposure to sterilization conditions that include gamma rays or ethylene oxide gas.

The conductive gel may be formed of any hydrophilic polymer that allows the conductive gel to function in accordance with the present disclosure. For example (but not by way of limitation), the conductive gel may be a polyacrylic acid gel, a povidone gel, or a cellulose gel. In addition, the conductive gel may comprise at least one of chitosan, alginate, agarose, methylcellulose, hyaluronan, collagen, laminin, matrigel, fibronectin, vitronectin, poly-1-lysine, proteoglycans, fibrin glue, gels made by decellularization of engineered and/or natural tissues, as well as any combinations thereof. Further, the conductive gel may comprise at least one of polyglycolic acid (PGA), polylactic acid (PLA), poly-caprolactone (PCL), polyvinyl alcohol (PVA), polyethylene glycol (PEG), methyl methacrylate, poly(methyl methacrylate) (PMMA), poly(2-hydroxyethyl methacrylate) (PolyHEMA), poly(glycerol sebacate), polyurethanes, poly(isopropylacrylamide), poly(N-isopropylacrylamide), or any combination thereof.

In certain non-limiting embodiments, the conductive gel comprises one or more of the following chemical and structural features/properties: a polymer chain length in a range of from about 1 nm to about 200 nm; a free salt present at a concentration in a range of from about 0.1 mM to about 1 M; a pH in a range of from about 6 to about 8; a volume resistivity of less than about 100 Ohm-in; a skin adhesion rate of at least about 100 g/inch; and a thickness in a range of from about 10 mil to about 50 mil.

In addition, given the prolonged exposure of the conductive gel composition to the patient's skin, the conductive gel should be optimized for use at body temperature (e.g., in a range of from about 34°C to about 44°C).

The polymer(s) of the conductive gel may be provided with any polymer chain length that allows the conductive gel composition(s) to function as described herein. For example (but not by way of limitation), the polymer chain length may be about 1 nm, about 2 nm, about 3 nm, about 4 nm, about 5 nm, about 6 nm, about 7 nm, about 8 nm, about 9 nm, about 10 nm, about 15 nm, about 20 nm, about 25 nm, about 30 nm, about 35 nm, about 40 nm, about 45 nm, about 50 nm, about 55 nm, about 60 nm, about 65 nm, about 70 nm, about 75 nm, about 80 nm, about 85 nm, about 90 nm, about 95 nm, about 100 nm, about 105 nm, about 110 nm, about 115 nm, about 120 nm, about 125 nm, about 130 nm, about 135 nm, about 140 nm, about 145 nm, about 150 nm, about 155 nm, about 160 nm, about 165 nm, about 170 nm, about 175 nm, about 180 nm, about 185 nm, about 190 nm, about 195 nm, about 200 nm, and above, as well as a range that combines any two of the above-referenced values (e.g., a range of from about 3 nm to about 175 nm, a range of from about 5 nm to about 150 nm, or a range of from about 10 nm to about 125 nm, a range of from about 15 nm to about 100 nm, etc.), and a range that combines two integers that fall between two of the above-referenced values (e.g., a range of from about 3 nm to about 157 nm, etc.).

In other non-limiting embodiments, the range of the polymer chain length is dependent upon the frequency(ies) of the alternating electric field. For example (but not by way of limitation), the range of the polymer chain length may be based upon a range of frequencies of the alternating electric field. Non-limiting examples include a range of from about 5 nm to about 50 nm when the alternating electric field has a frequency in a range of from about 50 kHz to about 150 kHz, a range of from about 50 nm to about 100 nm when the alternating electric field has a frequency in a range of from about 150 kHz to about 300 kHz, etc.

In certain non-limiting embodiments, the conductive gel has at least one of a decreased polymer chain length and an added free salt when compared to existing gel compositions; the decrease in polymer chain length and increase in free salt concentration further enhances the conductivity of the conductive gel while reducing the occurrence of skin irritation caused by the conductive gel. In a particular (but non-limiting) embodiment, the conductive gel comprises a free salt present via incorporation within the conductive gel or as one layer of a multi-layered gel (e.g., a bilayered gel). The term "free salt" refers to salt ions that are not incorporated as part of the polymerized chain structure but rather are floating substantially freely within the conductive gel and thus are a source of free ions that conduct electricity and thus reduce impedance.

When free salt is present in the conductive gel, the free salt may be any salt or other substance that serves as a source of free ions that are capable of floating substantially freely within the conductive gel, wherein the free ions serve to conduct electricity and thus reduce impedance. In certain particular (but non-limiting) embodiments, the free salt present in the conductive gel is a source of chloride ions, citrate ions, silver ions, iodide ions, etc., or any other ions that are known to be good conductors. Non-limiting examples of free salts that may be utilized in accordance with the present disclosure are salts that contain potassium (K), ammonium (NH4+), sodium (Na), nitrate, bicarbonate, and the like. Particular non-limiting examples of free salts that may be utilized in accordance with the present disclosure are NaCl, KCl, CaCl2, MgCl2, ZnCl2, silver iodide (Agl), silver dihydrogen citrate (SDC), sodium dihydrogen citrate, combinations thereof, and the like.

The free salt present in the gel may be provided with any concentration that allows the conductive gel compositions to function as described herein. For example (but not by way of limitation), the free salt concentration may be at least about 0.1 mM, about 0.5 mM, about 1 mM, about 2 mM, about 3 mM, about 4 mM, about 5 mM, about 6 mM, about 7 mM, about 8 mM, about 9 mM, about 10 mM, about 15 mM, about 20 mM, about 25 mM, about 30 mM, about 35 mM, about 40 mM, about 45 mM, about 50 mM, about 55 mM, about 60 mM, about 65 mM, about 70 mM, about 75 mM, about 80 mM, about 85 mM, about 90 mM, about 95 mM, about 100 mM, about 150 mM, about 200 mM, about 250 mM, about 300 mM, about 350 mM, about 476 mM, about 450 mM, about 576 mM, about 550 mM, about 676 mM, about 650 mM, about 776 mM, about 750 mM, about 876 mM, about 850 mM, about 976 mM, about 950 mM, about 1 M, or higher, as well as any range that combines any two of the above-referenced values (e.g., a range of from about 0.1 mM to about 100 mM, a range of from about 1 mM to about 50 mM, etc.), and a range that combines two integers that fall between two of the above-referenced values (e.g., a range of from about 12 mM to about 550 mM, etc.).

In other non-limiting embodiments, the free salt concentration is dependent upon the frequency(ies) of the alternating electric field. For example (but not by way of limitation), the range of the free salt concentration may be based upon a range of frequencies of the alternating electric field. Non-limiting examples include a range of from about 0.1 mM to about 50 mM when the alternating electric field has a frequency in a range of from about 50 kHz to about 150 kHz, a range of from about 50 mM to about 100 mM when the alternating electric field has a frequency in a range of from about 150 kHz to about 300 kHz, etc.

The conductive gel may be provided with any pH that does not damage the skin of a patient or cause chemical irritation of the skin upon prolonged exposure to the conductive gel. For example (but not by way of limitation), the conductive gel may have a pH of about 6, about 6.5, about 7, about 7.5, about 8, as well as a range formed from any of the above values (e.g., a range of from about 6 to about 8, a range of from about 6.5 to about 7.5, etc.).

The conductive gel may be provided with any level of volume resistivity that maximizes the conductivity of the gel. For example (but not by way of limitation), the conductive gel may have a volume resistivity of less than about 100 Ohm-in, less than about 95 Ohm-in, less than about 90 Ohm-in, less than about 85 Ohm-in, less than about 80 Ohm-in, less than about 75 Ohm-in, less than about 70 Ohm-in, less than about 65 Ohm-in, less than about 60 Ohm-in, less than about 55 Ohm-in, less than about 50 Ohm-in, less than about 45 Ohm-in, less than about 40 Ohm-in, less than about 35 Ohm-in, less than about 30 Ohm-in, less than about 25 Ohm-in, less than about 20 Ohm-in, less than about 15 Ohm-in, less than about 10 Ohm-in, or lower, as well as a range formed of any of the above values (e.g., a range of from about 10 Ohm-in to about 100 Ohm-in, etc.) and a range that combines two integers that fall between two of the above-referenced values (e.g., a range of from about 13 Ohm-in to about 96 Ohm-in, etc.).

The conductive gel may be provided with any skin adhesion rate that allows the conductive gel to function in accordance with the present disclosure. For example (but not by way of limitation), the skin adhesion rate of the gel may be at least about 100 g/inch, at least about 110 g/inch, at least about 120 g/inch, at least about 130 g/inch, at least about 140 g/inch, at least about 150 g/inch, at least about 160 g/inch, at least about 170 g/inch, at least about 180 g/inch, at least about 190 g/inch, at least about 200 g/inch, at least about 210 g/inch, at least about 220 g/inch, at least about 230 g/inch, at least about 240 g/inch, at least about 250 g/inch, at least about 260 g/inch, at least about 270 g/inch, at least about 280 g/inch, at least about 290 g/inch, at least about 300 g/inch, or higher, as well as a range of any of the above values (a range of from about 120 g/inch to about 300 g/inch, etc.), and a range that combines two integers that fall between two of the above-referenced values (e.g., a range of from about 115 g/inch to about 295 g/inch, etc.).

The conductive gel may be provided with any thickness that allows the conductive gel to function in accordance with the present disclosure. Non-limiting examples of thicknesses that may be utilized in accordance with the present disclosure include about 1 mil, about 5 mil, about 10 mil, about 15 mil, about 20 mil, about 25 mil, about 30 mil, about 35 mil, about 40 mil, about 45 mil, about 50 mil, about 55 mil, about 60 mil, about 65 mil, about 70 mil, about 75 mil, about 80 mil, about 85 mil, about 90 mil, about 95 mil, about 100 mil, or higher, as well as a range that combines any two of the above-referenced values (e.g., a range of from about 10 mil to about 50 mil, etc.), and a range that combines two integers that fall between two of the above-referenced values (e.g., a range of from about 12 mil to about 48 mil, etc.).

In certain particular (but non-limiting) embodiments, the conductive gel has a shelf life of at least about six months. For example (but not by way of limitation), the conductive gel has a shelf life of at least about 9 months or at least about 12 months.

In one embodiment, gel layer 158 is embedded into and throughout the foam layer 154. For example, if the foam layer 154 is a non-conductive foam, the embedded gel layer 158 may cause the foam layer 154 to be conductive between the gel layer 158 and the fabric layer 150, thereby allowing an electric signal, such as the TTField signal, to pass through the non-conductive foam. In one embodiment, the gel layer 158 may extend through the foam layer 154 to further contact the fabric layer 150.

In one embodiment, the electrode layer 162 is in contact with the gel layer 158. In one embodiment, as shown in FIG. 5, the electrode layer 162 is a constituent in the pad 70 such as described above with respect to FIG. 3. In this embodiment, each electrode element 104 is spatially disposed from each other and disposed between, and in contact with, both of the gel layer 158 and the durable topcoat layer 140. In some embodiments, the pad 70 may have a surface area less than that of the durable topcoat layer 140 and the gel layer 158, thereby causing at least a portion of the durable topcoat layer 140 to be in contact with a portion of the gel layer 158. A more detailed diagram of an embodiment of the electrode element 104 is shown in FIG. 6.

In one embodiment, the electrode elements 104 of the electrode layer 162 do not include a dielectric layer 192 as described below. In these embodiments, the electrode elements 104 may be in contact with the foam layer 154. Further, the electrode elements 104 may be in electrical contact with the foam layer 154 such that the foam layer 154 receives the TT Signal from the electric field generator 54.

In one embodiment, the array assembly 144 includes the compression layer 170. The compression layer 170 may be an exterior covering operable to cause a compression between the pad 70 and the patient's skin when the array assembly 144 is placed on the patient. In one embodiment, the compression layer 170 is a form of clothing, for example, a shirt, an undergarment, or a pants. In this embodiment, the fabric layer 150 may be sewn or otherwise affixed to the compression layer 170 such that, when the patient takes off or puts on clothing, the pad 70 does not substantially move relative to the compression layer 170. In one embodiment, the compression layer 170 is non-conductive.

In one embodiment, the compression layer 170 and the fabric layer 150 are sewn together to form a pocket. In this embodiment, the user, such as the patient or the healthcare provider, may place the pad 70 into the pocket. In some embodiments, the pocket may then be closed, such as by a button or hook and loop fastener, for example.

In one embodiment, the pad 70 further includes a removeable protection layer 176. The removeable protection layer 176 permits the pad 70 to be constructed separately from the compression layer 170 and the fabric layer 150 and placed together at a later time to form the array assembly 144. The step of placing the pad 70 between the fabric layer 150 and the compression layer 170 can be accomplished by the patient or healthcare provider at a point of care, or by a manufacturer of the array assembly 144.

In one embodiment, the dielectric layer 192 is provided within the pad 70. The dielectric layer 192 is constructed of one or more dielectric material and functions as an insulator. In some embodiments, the dielectric layer 192 includes a ceramic material. In other embodiments, the dielectric layer 192 is flexible. In some preferred embodiment, the dielectric layer 192 comprises poly(vinylidene fluoride-trifluoroethylene-chlorotrifluoroethylene) and/or poly(vinylidene fluoride-trifluoroethylene-1-chlorofluoroethylene). Those two polymers are abbreviated herein as "Poly(VDF-TrFE-CtFE)" and "Poly(VDF-TrFE-CFE)", respectively. These embodiments are particularly advantageous because the dielectric constant of these materials is on the order of 40. Because the TTFields are capacitively coupled through the dielectric layer 192, and because capacitance is inversely proportional to the thickness of the dielectric layer 192, the dielectric layer 192 is preferably as thin as possible (e.g., less than 10 µm or less than 5 µm). On the other hand, the dielectric layer 192 should not be too thin because that could impair manufacturability, compromise the layer's structural integrity, and risk dielectric breakdown when the AC signals are applied. In some preferred embodiments, the dielectric layer 192 has a thickness that is at least 1 µm. In some preferred embodiments the dielectric layer 192 is between 1-3 µm thick (e.g., about 2 µm), which provides a good balance between the parameters noted above. Preferably, the thickness of the dielectric layer 192 is uniform. But in alternative embodiments, the thickness could be non-uniform.

Optionally, ceramic nanoparticles may be mixed into the Poly(VDF-TrFE-CtFE) and/or Poly(VDF-TrFE-CFE). Optionally, these ceramic nanoparticles may comprise at least one of barium titanate and barium strontium titanate.

In alternative embodiments, instead of forming the dielectric layer 192 from Poly(VDF-TrFE-CtFE) and/or Poly(VDF-TrFE-CFE), a different polymer that provides a high dielectric constant, and/or a high level of capacitance may be used. The requirements for these different polymers are as follows: (1) at least one frequency between 50 kHz and 500 kHz, the polymer layer has a dielectric constant of at least 20; (2) the dielectric layer 192 has a thickness of less than 20 microns; and (3) the thickness of the dielectric layer 192 multiplied by its dielectric strength is at least 200 V. Example of alternative polymers that may be used in place of Poly(VDF-TrFE-CtFE) and/or Poly(VDF-TrFE-CFE) include the following: (1) ceramic nanoparticles mixed into at least one of Poly(VDF-TrFE), P(VDF-HFP), PVDF; and (2) barium titanate and/or barium strontium titanate ceramic nanoparticles mixed into at least one of Poly(VDF-TrFE), P(VDF-HFP), PVDF (where Poly(VDF-TrFE), P(VDF-HFP), and PVDF are, respectively poly(vinylidene fluoride-trifluoroethylene), poly(vinylidene fluoride-hexafluoropropylene) and poly(vinylidene fluoride)).

In some preferred embodiments, the thickness of the dielectric layer 192 is less than 10 µm, and in some preferred embodiments, the thickness of the dielectric layer 192 is less than 5 µm. In some preferred embodiments, the thickness of the dielectric layer 192 multiplied by its dielectric strength is at least 476 V. In some preferred embodiments, the dielectric layer 192 has a dielectric constant of at least 20 measured at 200 kHz.

Referring now to FIG. 6, shown therein is a cross-sectional view of an exemplary embodiment of an electrode assembly 180 constructed in accordance with the present disclosure. As shown in FIG. 6, the electrode assembly 180 comprises at least one electrode element 104. The electrode element 104 comprises at least one conductor 188, and the dielectric layer 192, and, optionally, may further comprise at least one non-conducting layer 184 (which, for example, may be the durable topcoat layer, 140), as shown in FIG. 6. In one embodiment, the dielectric layer 192 is a high capacitance layer. In one embodiment, the electrode assembly 180 further includes at least one optional opening 196 disposed at least partially therethrough. In one embodiment, the electrode assembly 180 may be constructed as disclosed in US Patent No. 7,089,054 entitled "Apparatus and Method for Treating a Tumor or the Like".

Referring now to FIG. 7, shown therein is a top view of an exemplary embodiment of a fabric layer 150 constructed in accordance with the present disclosure. Generally, the fabric layer 150 comprises nonconductive threads 220 connected to one or more conductive thread 224a-n. For example, the nonconductive threads 220 can be interwoven with the one or more conductive thread 224a-n shown in FIG. 7 as conductive thread 224a, conductive thread 224b, and conductive thread 224c. Each of the conductive threads 224a-n can be woven with the nonconductive threads 220. In one embodiment, the conductive threads 224a-n extend across the fabric layer 150 so as to create a substantially continuous conductive region across the fabric layer 150. The fabric layer 150 may include a plurality of perforations 228, which may be formed by a space between adjacently disposed nonconductive threads 220 and/or conductive threads 224 of the fabric layer 150.

In one embodiment, the fabric layer 150 covers at least a portion of the skin-facing surface 156 of the foam layer 154. In other embodiments, the fabric layer 150 covers the entirety of the skin-facing surface 156 of the foam layer 154. Optionally, the removable protection layer 176 may reside between the fabric layer 150 and the skin-facing surface 156 of the foam layer 154 (see FIG. 5).

The nonconductive threads 220 and the conductive threads 224 can be connected together in a woven format or a non-woven format. In the non-woven format, the nonconductive threads 220 and the conductive threads 224 can be bonded together by entangling the nonconductive threads 220 and the conductive threads 224 mechanically, thermally or chemically. The nonconductive threads 220 and the conductive threads 224 may be bonded together in a manner so that the fabric layer 150 is flat or tufted. The fabric layer 150 may be constructed of any type of fabric having conductive threads 224 and optionally nonconductive threads 220, such as woven fabric, non-woven fabric, or knit fabric, or any combination thereof.

In one embodiment, material for the nonconductive threads 200 may be selected from any non-conductive material having desirable properties such as, but not limited to, strong biocompatibility and low reactivity with other layers or components of the pad 70 as shown in FIG. 5.

In one embodiment, the fabric layer 150 has an adhesive property such that the fabric layer 150 has a propensity to, when placed at a particular location on a patient, stay at that particular location. In some embodiments, the fabric layer 150, in conjunction with the compression layer 170, is sized to fit tightly and in an encircling, and form fitting manner onto a portion of the patient's body so as to maintain electrical conductivity between the fabric layer 150 and the patient's skin. The fabric layer 150, in conjunction with the compression layer 170, can be formed into a configuration suitable for use as a tight-fitting garment or a brace. For example, the compression layer 170 can be formed into a tubular configuration and used as a knee brace which surrounds the patient's knee when the fabric layer 150 is placed onto the patient's knee. In another embodiment, the compression layer 170 can be formed into a beltlike configuration having an attachment mechanism on one end, (e.g., buckle, hook and loop (Velcro) or the like), suitable for use as a back brace with the fabric layer 150 between at least a portion of the compression layer 170 and the patient. The fabric layer 150 and the compression layer 170 may be sewn together forming a pocket operable to receive the pad 70. The pad 70 may thus be held in place against the patient.

In one embodiment, the conductive thread 224 may be constructed of a conductive material able to be bonded to and/or woven with the nonconductive threads 220 and able to withstand multiple distortions without compromising conductivity along the conductive thread 224. For example, the conductive thread 224 may be selected from any conductive material having desirable properties such as, but not limited to, high conductivity, strong biocompatibility, and low reactivity with other layers or components of the array assembly 144. In one embodiment, the conductive thread 224 is selected from a conductive material made from, bonded with, or coated with one or more of silver, copper, tin, aluminum, titanium, platinum, carbon, an alloy thereof, and/or some combination thereof. In one embodiment, the conductive thread 224 is of a thickness sufficient to support conductivity of a voltage and an amperage suitable to generate TTFields and sufficient to cause flexible contouring of the array assembly 144.

FIG. 7 depicts three conductive threads 224a-c; however, it is understood that the number of conductive threads 224 within the fabric layer 150 could be greater than or lesser than three. Further, while the conductive threads 224a-n are shown as being substantially evenly, spatially disposed within the fabric layer 150 along with nonconductive threads 220, it is understood that the conductive threads 224a-n may be threaded, sewn, or otherwise disposed between nonconductive threads 220 of the fabric layer 150. In one embodiment, the fabric layer 150 does not include the nonconductive threads 220.

Referring now to FIG. 8, shown therein is a diagram of an exemplary embodiment of the foam layer 154 constructed in accordance with the present disclosure. As described above, the foam layer 154 may include a solid continuous phase material 239 and one or more pocket 240 interspersed throughout the solid continuous phase material 239. Each of the one or more pocket 240 may be on a surface of the foam layer 154 such as the skin-facing surface 156 as shown by pockets 240a-c, or internal to the foam layer 254 as shown by pocket 240d. The pockets 240a-c on the skin-facing surface 156 may be partially exposed, such as the pocket 240c, semi-exposed, such as the pocket 240b, or mostly exposed, such as the pocket 240a.

In one embodiment, each pocket 240 has a diameter of between about 80 mils and about 120 mil. In some embodiments, one or more pocket 240 has a diameter greater than 120 mil.

Further shown in FIG. 8 is the gel layer 158 when the gel layer 158 is disposed on the skin-facing surface 156 of the foam layer 154. As shown the gel layer 158 may be absorbed or adsorbed by the foam layer 154 such that pockets 240 internal to the foam layer 154, such as the pocket 240d, is at least partially filled with gel from the gel layer 158. Additionally, the pocket 240a and the pocket 240c are shown as having gel from the gel layer 158. However, in some instances, due to manufacturing or properties of the gel layer 158, not all pockets 240 may be filled with the gel from the gel layer 158 as shown by the pocket 240b. In some cases, one or more pocket 240 may be partially filled by gel from the gel layer 158.

In one embodiment, the gel layer 158 is applied to the foam layer 154 as a liquid gel, such as a liquid hydrogel, and is cured, or polymerized, after it has been applied to the foam layer 154.

Referring now to FIG. 9, shown therein is a cross section of an exemplary embodiment of an array assembly 250 constructed in accordance with the present disclosure. The array assembly 250 may be constructed similar to the array assembly 144 (see FIG. 5, and with similar labelling) with the exception that the pad 70 having the electrode layer 162 formed by the electrode elements 104 is a pad 70c having the electrode layer 162 formed by the electrode element 136.

Referring now to FIG. 10, shown therein is a cross-sectional diagram of an exemplary embodiment of a pad 70d constructed in accordance with the present disclosure. The pad 70d generally comprises the second end 66 of the conductive lead 58 connected to the foam layer 154. In this embodiment, the foam layer 154 is a conductive foam that receives the TTField signals from the electric field generator 54 which is operable to generate the TTFields. One or more of the pad 70a and the pad 70b may be substituted with the pad 70d.

In this embodiment, the pad 70d may further include the gel layer 158, which may be disposed on the skin-facing surface 156 of the foam layer 154. As discussed in more detail above, the gel layer 158 may be absorbed or adsorbed into the foam layer 154 such that the foam layer 154 includes at least a portion of the gel layer 158 in the plurality of pockets 240 within the foam layer 154.

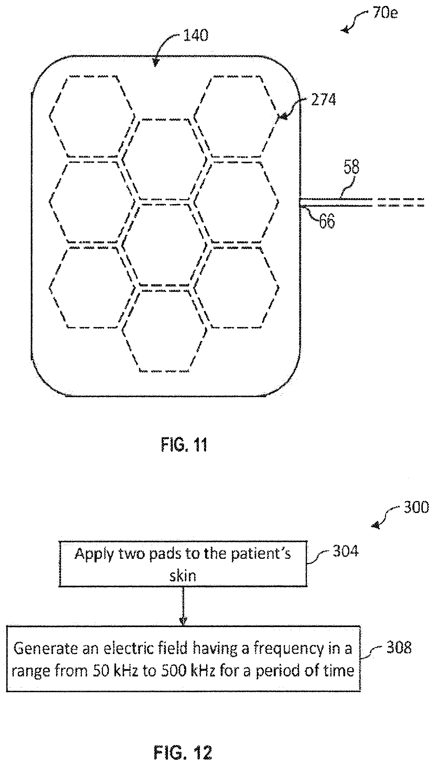

Referring now to FIG. 11, shown therein is diagram of a top view of an exemplary embodiment of a pad 70e constructed in accordance with the present disclosure. The pad 70e may be constructed similar to one of the pad 70, pad 70c, or pad 70d with the exception that the foam layer 154 is shaped into one or more polygon shape 274. The polygon shape 274 shown in FIG. 11 is shown as a regular, equilateral hexagon, however, in other embodiments, the foam layer 154 may be shaped into other polygons with a fewer or a greater number of sides than six sides. For example, the foam layer 154 may be shaped into a triangle, square, or pentagon, for example. In other embodiments, the polygon shape 274 may be a heptagon, octagon, nonagon, decagon, or other polygon with an even greater number of sides.

In one embodiment, the polygon shape 274 may be repeated one or more times within the pad70e. In some embodiments, the pad 70e may include a first polygon shape 274 having a first shape and a second polygon shape having a second shape, where the first shape and the second shape are different.

In one embodiment, each polygon shape 274 is spatially disposed and separated from each other by a non-conductive, or dielectric, material. In some embodiments, however, each polygon shape 274 is spatially disposed and separated from each other by a conductive material.

In one embodiment, each electrode element 104 of the pad 70 is disposed within a polygon shape 274. In this embodiment, each electrode element 104 may be disposed at a center of the polygon shape 274 or may be disposed a particular distance from another electrode element 104, or both.

In one embodiment, each polygon shape 274 is sized such that the pad 70e, when placed on a patient's head, contours to that patient's head. In some embodiments, each polygon shape 274 has a diameter greater than about 1 inch and lesser than about 2 inches. In other embodiments, each polygon shape has a diameter greater than about 2 inches and lesser than about 4 inches. In one embodiment, all polygon shapes 274 are about the same size, whereas in other embodiments, one or more polygon shape 274 are not the same size.

In one embodiment, shown in FIG. 11, each polygon shape 274 is disposed evenly within the pad 70e, however, in other embodiments, each polygon shape 274 is not disposed evenly within the pad 70e. Additionally, in some embodiments, the polygon shape 274 is not a regular, or equilateral, polygon, but may be a polygon where two or more sides are unequal in length, or, alternatively or additionally, may have rounded vertices. Additionally, in some embodiments, the polygon shape 274 includes a concave polygon instead of a convex polygon shape.

Referring now to FIG. 12, shown therein is an exemplary embodiment of a process 300 of using the electronic apparatus 50 to apply a TTField to a patient. The process 300 generally comprises the steps of: applying two pads to the patient's skin (step 304) and generating an alternating electric field (TTField) having a frequency in a range of from about 50 kHz to about 500 kHz for a period of time (step 308).

In one embodiment, the step of applying two pads to the patient's skin (step 304) includes selecting one or more of the pad 70, the pad 70a, the pad 70b, the pad 70c, the pad 70d or the pad 70e, and applying the selected pads to the patient's skin.

In one embodiment, the step of applying two pads to the patient's skin (step 304) includes applying two or more pads to the patient's skin. In some embodiments, the number of pads 70 applied to a patient's skin is determined by a number of pads 70 needed to apply a TTField having a therapeutic benefit as determined by the user, such as by a medical professional.

The step of applying two pads (step 304) may be performed by the user. In one embodiment, before applying the selected pads to the patient's skin, the patient's skin may need to be cleaned (e.g., such as but not limited to, cleansing of the skin of foreign matter or biological matter and shaving of the skin, if necessary).

The step of generating an alternating electric field (TTField) (step 308) may be performed by the electric field generator 54 and may be instantiated by an operation performed by the user or the control box 74. In one embodiment, step 308 may be performed more than one time and the period of time for which the step 308 is performed a first time may be the same as or different from the period of time for which the step 308 is performed a second time (or other period(s) of time beyond the second time).

In some embodiments, step 308 is only performed once before the process 300 is repeated. There may be a time period between each time the process 300 is repeated. Each time the process 300 is repeated, the time period may be the same as or different from the previous time period. Each time the process 300 is repeated, the selected pads may be placed in the same or a different position on the patient's skin.

In one embodiment, prior to generating an alternating electric field (TTField) having a frequency in a range of from about 50 kHz to about 500 kHz for a period of time (step 308), the user connects, or electrically couples, the selected pads to the electric field generator 54.

From the above description, it is clear that the inventive concepts disclosed and claimed herein are well adapted to carry out the objects and to attain the advantages mentioned herein, as well as those inherent in the invention. While exemplary embodiments of the inventive concepts have been described for purposes of this disclosure, it will be understood that numerous changes may be made which will readily suggest themselves to those skilled in the art and which are accomplished within the scope of the appended claims.

The foregoing description provides illustration and description, but is not intended to be exhaustive or to limit the inventive concepts to the precise form disclosed. Modifications and variations are possible in light of the above teachings or may be acquired from practice of the methodologies set forth in the present disclosure.

Similarly, although each illustrative embodiment listed above may directly depend on only one other illustrative embodiment, the disclosure includes each illustrative embodiment in combination with every other illustrative embodiment in the set of illustrative embodiments for each mode of the inventive concepts disclosed herein.

No element, act, or instruction used in the present application should be construed as critical or essential to the invention unless explicitly described as such outside of the preferred embodiment. Further, the phrase "based on" is intended to mean "based, at least in part, on" unless explicitly stated otherwise.

## Claims

1. A system for delivering tumor treating fields, TTFields, to a body of a subject, the system comprising:
an electric field generator (54) configured to generate an electrical signal having an alternating current waveform at a frequency in a range from 50 kHz to 500 kHz;
a first conductive lead (58; 58a) electrically coupled to the electric field generator (54), the first conductive lead (58; 58a) being configured to carry the electrical signal;
a first pad (70; 70a; 70c; 70d; 70e) coupled to the first conductive lead (58; 58a), the first pad (70; 70a; 70c; 70d; 70e) having a foam (154) and a first conductive gel element (158);
a second conductive lead (58; 58b) electrically coupled to the electric field generator (54), the second conductive lead (58; 58b) being configured to carry the electrical signal; and
a second pad (70; 70b; 70c; 70d; 70e) coupled to the second conductive lead (58; 58b), the second pad (70; 70b; 70c; 70d; 70e) having an electrode element (104; 136) and receiving the electrical signal from the second conductive lead (58; 58b), the electrode element (104; 136) being connected to a second conductive gel element (158);
**characterised by**:
the foam (154) being a conductive foam (154) and receiving the electrical signal from the first conductive lead (58; 58a);
the conductive foam (154) having a solid continuous phase material (239) being at least one of constructed of a conductive material or having a conductive material attached, absorbed or adsorbed to the solid continuous phase material (239) and defining a plurality of pockets (240; 240a-d) interspersed throughout the solid continuous phase material (239); and
the first conductive gel element (158) being attached, absorbed or adsorbed to the conductive foam (154).

2. The system of claim 1, wherein the conductive material of the solid continuous phase material (239) includes silver.

3. The system of claim 1 or 2, wherein the first pad (70; 70a; 70c; 70d; 70e) further comprises at least one electrode layer (162) and a dielectric layer (192), the dielectric layer (192) being positioned between the at least one electrode layer (162) and the conductive foam (154).

4. The system of claim 3, wherein the at least one electrode layer (162) comprises a plurality of spatially-disposed electrode elements (104; 136).

5. The system of claim 3, wherein the dielectric layer (192) comprises a plurality of spatially-disposed dielectric elements, optionally including a flexible polymer material.

6. The system of claim 5, wherein the dielectric elements include a ceramic material.

7. The system of claim 1 or 2, wherein the first pad (70; 70a; 70c; 70d; 70e) comprises at least one electrode layer (162) electrically coupled with the conductive foam (154) without a dielectric material positioned between the at least one electrode layer (162) and the conductive foam (154).

8. The system of any of claims 1, 2 and 7, wherein the at least one electrode layer (162) includes an electrode element (104; 136) in contact with the conductive foam (154).

9. The system of any of claims 1 to 8, further comprising:
a blocking capacitor (82a) in circuit with the conductive foam (154) and operable to prevent an occurrence of direct current within the conductive foam (154).

10. The system of claim 9, wherein the blocking capacitor (82a) is integrated within at least one of the electric field generator (54) and the first conductive lead (58; 58a).

11. The system of any of claims 1 to 10, wherein the conductive foam (154) includes a skin-facing surface (156), and the first pad (70; 70a; 70c; 70d; 70e) further comprises a conductive cloth (150) covering the skin-facing surface (156) of the conductive foam (154).

12. The system of any of claims 1 to 11, further comprising:
one or more temperature sensors (78) configured to measure a temperature of the first pad (70; 70a; 70c; 70d; 70e).

13. The system of claim 12, further comprising:
a control box (74) configured to monitor the one or more temperature sensors (78) and turn off the electric field generator (54) if the temperature exceeds a comfortability threshold, optionally the comfortability threshold is selected to be a value between about 39 degrees Celsius and about 42 degrees Celsius.

14. The system of any of claims 1 to 13, wherein the first conductive gel element (158) is positioned within at least some of the pockets (240; 240a, c).

15. The system of any of claims 1 to 14, wherein the conductive foam (154) has a skin-facing surface (156), and the first conductive gel element (158) is attached, absorbed or adsorbed on the skin-facing surface (156) of the conductive foam (154).

## Patentansprüche

1. System zur Abgabe von Tumorbehandlungsfeldern, TTFields, an den Körper eines Subjekts, wobei das System umfasst:
einen elektrischen Feldgenerator (54), der so konfiguriert ist, dass er ein elektrisches Signal mit einer Wechselstromwellenform bei einer Frequenz in einem Bereich von 50 kHz bis 500 kHz erzeugt;
eine erste leitfähige Leitung (58; 58a), die elektrisch mit dem elektrischen Feldgenerator (54) gekoppelt ist, wobei die erste leitfähige Leitung (58; 58a) so konfiguriert ist, dass sie das elektrische Signal überträgt;
eine erste Elektrode (70; 70a; 70c; 70d; 70e), die mit der ersten leitfähigen Leitung (58; 58a) gekoppelt ist, wobei die erste Elektrode (70; 70a; 70c; 70d; 70e) einen Schaum (154) und ein erstes leitfähiges Gelelement (158) aufweist;
eine zweite leitfähige Leitung (58; 58b), die elektrisch mit dem elektrischen Feldgenerator (54) gekoppelt ist, wobei die zweite leitfähige Leitung (58; 58b) so konfiguriert ist, dass sie das elektrische Signal überträgt; und
eine zweite Elektrode (70; 70b; 70c; 70d; 70e), die mit der zweiten leitfähigen Leitung (58; 58b) gekoppelt ist, wobei die zweite Elektrode (70; 70b; 70c; 70d; 70e) ein Elektrodenelement (104; 136) aufweist und das elektrische Signal von der zweiten leitfähigen Leitung (58; 58b) empfängt, wobei das Elektrodenelement (104; 136) mit einem zweiten leitfähigen Gel-Element (158) verbunden ist;
**dadurch gekennzeichnet, dass**:
der Schaum (154) ein leitfähiger Schaum (154) ist und das elektrische Signal von der ersten leitfähigen Leitung (58; 58a) empfängt;
wobei der leitfähige Schaum (154) ein festes Material mit kontinuierlicher Phase (239) aufweist, das zumindest entweder aus einem leitfähigen Material besteht oder ein leitfähiges Material aufweist, das an dem festen Material mit kontinuierlicher Phase (239) angebracht, absorbiert oder adsorbiert ist, und eine Vielzahl von Taschen (240; 240a-d) definiert, die über das feste, kontinuierliche Phasenmaterial (239) verteilt sind; und
wobei das erste leitfähige Gelelement (158) an dem leitfähigen Schaum (154) angebracht, absorbiert oder adsorbiert ist.

2. System nach Anspruch 1, wobei das leitfähige Material des festen Materials mit kontinuierlicher Phasenmaterials (239) Silber enthält.

3. System nach Anspruch 1 oder 2, wobei die erste Elektrode (70; 70a; 70c; 70d; 70e) ferner mindestens eine Elektrodenschicht (162) und eine dielektrische Schicht (192) umfasst, wobei die dielektrische Schicht (192) zwischen der mindestens einen Elektrodenschicht (162) und dem leitfähigen Schaum (154) angeordnet ist.

4. System nach Anspruch 3, wobei die mindestens eine Elektrodenschicht (162) eine Vielzahl von räumlich angeordneten Elektrodenelementen (104; 136) umfasst.

5. System nach Anspruch 3, wobei die dielektrische Schicht (192) eine Vielzahl von räumlich angeordneten dielektrischen Elementen umfasst, die optional ein flexibles Polymermaterial enthalten.

6. System nach Anspruch 5, wobei die dielektrischen Elemente ein keramisches Material enthalten.

7. System nach Anspruch 1 oder 2, wobei die erste Elektrode (70; 70a; 70c; 70d; 70e) mindestens eine Elektrodenschicht (162) umfasst, die elektrisch mit dem leitfähigen Schaum (154) gekoppelt ist, ohne dass ein dielektrisches Material zwischen der mindestens einen Elektrodenschicht (162) und dem leitfähigen Schaum (154) angeordnet ist.

8. System nach einem der Ansprüche 1, 2 und 7, wobei die mindestens eine Elektrodenschicht (162) ein Elektrodenelement (104; 136) enthält, das in Kontakt mit dem leitfähigen Schaum (154) steht.

9. System nach einem der Ansprüche 1 bis 8, ferner umfassend:
einen Sperrkondensator (82a) im Schaltkreis mit dem leitfähigen Schaum (154), der dazu dient, das Auftreten von Gleichstrom innerhalb des leitfähigen Schaums (154) zu verhindern.

10. System nach Anspruch 9, bei dem der Sperrkondensator (82a) in mindestens einen des elektrischen Feldgenerators (54) und die erste leitfähige Leitung (58; 58a) integriert ist.

11. System nach einem der Ansprüche 1 bis 10, wobei der leitfähige Schaumstoff (154) eine der Haut zugewandte Oberfläche (156) aufweist und die erste Elektrode (70; 70a; 70c; 70d; 70e) ferner ein leitfähiges Tuch (150) umfasst, dass die der Haut zugewandte Oberfläche (156) des leitfähigen Schaumstoffs (154) bedeckt.

12. System nach einem der Ansprüche 1 bis 11, ferner umfassend:
einen oder mehrere Temperatursensoren (78), die so konfiguriert sind, dass sie eine Temperatur der ersten Elektrode (70; 70a; 70c; 70d; 70e) messen.

13. System nach Anspruch 12, ferner umfassend:
eine Steuerbox (74), die so konfiguriert ist, dass sie den einen oder die mehreren Temperatursensoren (78) überwacht und den elektrischen Feldgenerator (54) ausschaltet, wenn die Temperatur einen Schwellenwert für die Behaglichkeit überschreitet, wobei der Schwellenwert für die Behaglichkeit optional auf einen Wert zwischen etwa 39 Grad Celsius und etwa 42 Grad Celsius festgelegt ist.

14. System nach einem der Ansprüche 1 bis 13, wobei das erste leitfähige Gelelement (158) in mindestens einigen der Taschen (240; 240a, c) angeordnet ist.

15. System nach einem der Ansprüche 1 bis 14, wobei der leitfähige Schaumstoff (154) eine der Haut zugewandte Oberfläche (156) aufweist und das erste leitfähige Gelelement (158) an welcher der Haut zugewandten Oberfläche (156) des leitfähigen Schaumstoffs (154) angebracht, absorbiert oder adsorbiert ist.

## Revendications

1. Système pour admnistrer des champs de traitement de tumeur, TTFields, au corps d'un sujet, le système comprenant :
un générateur de champ électrique (54) configuré pour générer un signal électrique présentant une forme d'onde de courant alternatif à une fréquence dans une plage de 50 kHz à 500 kHz ;
un premier fil conducteur (58 ; 58a) couplé électriquement au générateur de champ électrique (54), le premier fil conducteur (58 ; 58a) étant configuré pour transporter le signal électrique ;
un premier plot (70 ; 70a ; 70c ; 70d ; 70e) couplé au premier fil conducteur (58 ; 58a), le premier plot (70 ; 70a ; 70c ; 70d ; 70e) présentant une mousse (154) et un premier élément de gel conducteur (158) ;
un second fil conducteur (58 ; 58b) couplé électriquement au générateur de champ électrique (54), le second fil conducteur (58 ; 58b) étant configuré pour transporter le signal électrique ; et
un second plot (70 ; 70b ; 70c ; 70d ; 70e) couplé au second fil conducteur (58 ; 58b), le second plot (70 ; 70b ; 70c ; 70d ; 70e) présentant un élément d'électrode (104 ; 136) et recevant le signal électrique provenant du second fil conducteur (58 ; 58b), l'élément d'électrode (104 ; 136) étant relié à un second élément de gel conducteur (158) ;
le premier plot (70 ; 70a ; 70c ; 70d ; 70e) *présentant* une mousse conductrice (154) et un premier élément de gel conducteur (158),
**caractérisé par** :
la mousse (154) étant une mousse conductrice (154) et recevant le signal électrique provenant du premier fil conducteur (58 ; 58a) ;
la mousse conductrice (154) présentant un matériau de phase continue solide (239) qui est construit à partir d'un matériau conducteur et/ou présentant un matériau conducteur attaché, absorbé ou adsorbé au matériau de phase continue solide (239) et définissant une pluralité de poches (240 ; 240a-d) intercalées dans tout le matériau de phase continue solide (239) ; et
le premier élément de gel conducteur (158) étant attaché, absorbé ou adsorbé à la mousse conductrice (154).

2. Système selon la revendication 1, dans lequel le matériau conducteur du matériau de phase continue solide (239) inclut de l'argent.

3. Système selon la revendication 1 ou 2, dans lequel le premier plot (70 ; 70a ; 70c ; 70d ; 70e) comprend en outre au moins une couche d'électrode (162) et une couche diélectrique (192), la couche diélectrique (192) étant positionnée entre l'au moins une couche d'électrode (162) et la mousse conductrice (154).

4. Système selon la revendication 3, dans lequel l'au moins une couche d'électrode (162) comprend une pluralité d'éléments d'électrode disposés spatialement (104 ; 136).

5. Système selon la revendication 3, dans lequel la couche diélectrique (192) comprend une pluralité d'éléments diélectriques disposés spatialement, incluant éventuellement un matériau polymère flexible.

6. Système selon la revendication 5, dans lequel les éléments diélectriques incluent un matériau céramique.

7. Système selon la revendication 1 ou 2, dans lequel le premier plot (70 ; 70a ; 70c ; 70d ; 70e) comprend au moins une couche d'électrode (162) couplée électriquement à la mousse conductrice (154) sans matériau diélectrique positionné entre l'au moins une couche d'électrode (162) et la mousse conductrice (154).

8. Système selon l'une des revendications 1, 2 et 7, dans lequel l'au moins une couche d'électrode (162) inclut un élément d'électrode (104 ; 136) en contact avec la mousse conductrice (154).

9. Système selon l'une des revendications 1 à 8, comprenant en outre :
un condensateur de blocage (82a) en circuit avec la mousse conductrice (154) et pouvant fonctionner pour empêcher l'apparition d'un courant continu dans la mousse conductrice (154).

10. Système selon la revendication 9, dans lequel le condensateur de blocage (82a) est intégré dans au moins l'un du générateur de champ électrique (54) et du premier fil conducteur (58 ; 58a).

11. Système selon l'une des revendications 1 à 10, dans lequel la mousse conductrice (154) inclut une surface tournée vers la peau (156), et le premier plot (70 ; 70a ; 70c ; 70d ; 70e) comprend en outre un tissu conducteur (150) couvrant la surface tournée vers la peau (156) de la mousse conductrice (154).

12. Système selon l'une des revendications 1 à 11, comprenant en outre :
un ou plusieurs capteurs de température (78) configurés pour mesurer une température du premier plot (70 ; 70a ; 70c ; 70d ; 70e).

13. Système selon la revendication 12, comprenant en outre :
un boîtier de commande (74) configuré pour surveiller le ou les plusieurs capteurs de température (78) et éteindre le générateur de champ électrique (54) si la température dépasse un seuil de confort, éventuellement le seuil de confort est sélectionné pour être une valeur comprise entre environ 39 degrés Celsius et environ 42 degrés Celsius.

14. Système selon l'une des revendications 1 à 13, dans lequel le premier élément de gel conducteur (158) est positionné dans au moins certaines des poches (240 ; 240a, c).

15. Système selon l'une des revendications 1 à 14, dans lequel la mousse conductrice (154) présente une surface tournée vers la peau (156), et le premier élément de gel conducteur (158) est fixé, absorbé ou adsorbé sur la surface tournée vers la peau (156) de la mousse conductrice (154).
